Europäisches Patentamt

European Patent Office

Office européen des brevets

(19)

(11) Veröffentlichungsnummer: **0 229 614 B1**

(12) # EUROPÄISCHE PATENTSCHRIFT

(45) Veröffentlichungstag der Patentschrift: **11.11.92**

(51) Int. Cl.5: **C12P 1/00**, B01J 23/00

(21) Anmeldenummer: **87100040.2**

(22) Anmeldetag: **03.01.87**

(54) Verfahren zur selektiven Regenerierung von Mediatoren und Katalysator dafür.

(30) Priorität: **08.01.86 DE 3600274**

(43) Veröffentlichungstag der Anmeldung:
**22.07.87 Patentblatt 87/30**

(45) Bekanntmachung des Hinweises auf die
Patenterteilung:
**11.11.92 Patentblatt 92/46**

(84) Benannte Vertragsstaaten:
**AT BE CH DE ES FR GB IT LI NL SE**

(56) Entgegenhaltungen:
**EP-A- 0 118 928**
**EP-A- 0 197 403**
**FR-A- 2 252 131**

**JOURNAL OF SOLID-PHASE BIOCHEMISTRY,
Band 5, Nr. 4, 1980, Seiten 269-282, Plenum
Publishing Corp.; P. MEIER et al.: "Micellar
solubilization of biopolymers in hydrocarbon
solvents. II. The case of horse liver alcohol
dehydrogenase"**

**CHEMICAL ABSTRACTS, Band 105, Nr. 5, August 1986, Seite 612, Zusammenfassung Nr.
41296f, Columbus, Ohio, US; & JP-A-61 56
090 (T. MATSUNAGA) 20-03-1986**

**CHEMICAL ABSTRACTS, Band 85, Nr. 6, August 1976, Seite 322, Zusammenfassung Nr.
37662y, Columbus, Ohio, US; & JP-A-76 22
690 (NIPPON SHOKUBAI KAGAKU KOGYO
CO., LTD) 23-02-1976**

(73) Patentinhaber: **BASF Aktiengesellschaft
Carl-Bosch-Strasse 38
W-6700 Ludwigshafen(DE)**

(72) Erfinder: **Simon, Helmut, Prof. Dr.
Egilbertstrasse 31
W-8050 Freising(DE)**
Erfinder: **Thanos, Jordanes, Dr.
Silcherstrasse 3
W-8000 Muenchen(DE)**

## Beschreibung

Die Erfindung betrifft ein Verfahren zur katalytischen Hydrierung von Mediatoren (Elektronenüberträger) mit Hilfe spezieller Katalysatoren

Es ist bereits bekannt, Elektronen auf biochemische Redox-Enzyme mit Hilfe von Mediatoren - auch als Elektronenüberträger bezeichnet - zu übertragen. Hierzu müssen die Mediatoren kontinuierlich reduziert werden. Das geschah bislang mit Wasserstoff in Gegenwart einer Hydrogenase, Formiat in Gegenwart einer Viologen-abhängigen Formiatdehydrogenase (Angew. Chem. 97, 541, 617 (1985), Angew. Chem. Int. Ed. Engl. 24, 539, 617 (1985) oder elektrochemisch (EP-OS 99 517). Die beiden ersten Verfahren haben den Nachteil, daß ein zweites, in der Regel empfindliches Enzym verwendet werden muß. Die elektrochemische Reduktion erfordert einen beträchtlichen apparativen Aufwand.

Die Reduktion von Viologenen kann auch mit Zink oder Dithionit (J. Amer. Chem. Soc. 107, 2632 (1985)) oder mit Wasserstoff und Platin auf Asbest erfolgen (J. Biol. Chem. 249, 1572 (1974)). Diese Verfahren können jedoch nicht kontinuierlich durchgeführt werden, da das Zink bzw. Dithionit verbraucht werden und das Methylviologen mit Platin nicht beliebig oft regeneriert werden kann, da das Methylviologen relativ schnell zerstört wird. Außerdem stören Metallkationen und Sulfitanionen, die sich während der Reaktion anhäufen, die Enzymreaktionen. Es wurde nun gefunden, daß sich Mediatoren wie das Methylviologen mit bestimmten Katalysatoren sehr gut hydrieren lassen.

Gegenstand der Erfindung sind ein Verfahren zur selektiven Regenerierung von Mediatoren durch katalytische Hydrierung, dadurch gekennzeichnet, daß man als Katalysator einen mit Hilfe von Tensiden modifizierten Metallkatalysator verwendet, sowie die Verwendung diese modifizierten Katalysatoren zur Durchführung biochemischer Reduktionen.

Die Hydrierung wird unter Wasserstoffatmosphäre bei Normaldruck oder leichtem Überdruck unter Ausschluß von Sauerstoff durchgeführt. Die Temperatur und der pH-Wert der Umsetzung hängen von dem verwendeten Enzym ab. In der Regel wird bei Temperaturen zwischen 10 und 90°C, vorzugsweise zwischen 25 - 40°C, und bei pH-Werten zwischen 3 und 10, vorzugsweise 5 - 8 gearbeitet.

Als Mediatoren kommen folgende Substanzen in Betracht:

1. Viologenfarbstoffe, z.B. Methylviologen, Benzylviologen, Carboxamidmethylviologen, Diquat,
2. Anthrachinon und andere Chinon-Farbstoffe, z.B. Phenosafranin, Methylenblau, Anthrachinonsulfonsäuren,
3. Triphenylmethan-Farbstoffe, z.B. Methylviolett, Kristallviolett,
4. Phthalocyanine, z.B. Fe-, Cu- oder Co-Phthalocyanin
5. Methinfarbstoffe, z.B. Astraphloxin,
6. Pyrrolfarbstoffe oder Porphyrinderivate, z.B. Metall-Chelatkomplexe dieser Verbindungen,
7. Pteridine und Pteridone
8. Flavine, z.B. Acriflavin, Lumiflavin
9. Metallkomplexe der Metalle der 6., 7., 8. Nebengruppe, z.B. $Ru(L_2L'_2)^{++}$ [L = 1,10 Phenanthrolin, 2,2-Bipyridyl oder 5-Nitro-1,10-phenanthrolin; $L'$ = Pyridin oder 4-Methylpyridin], 1,1'-Bis(hydroxymethyl)ferrocene bzw. Ferrocen-Monocarbonsäuren.

Unter diesen ist die 1. Gruppe, insbesondere das Methyl- und Benzylviologen, bevorzugt.

Als Tenside eigenen sich solche, die Metalloberflächen gut benetzen, d.h. insbesondere solche, deren Kohlenwasserstoffreste in hohem Maß fluoriert sind. Insbesondere kommen Fluor-haltige kationische Tenside in Betracht, wie sie unter der Bezeichnung (R)Zonyl erhältlich sind. Die beste Wirkung zeigte das Tensid (R)Zonyl FSC, für das die Struktur $[F(CF_2-CF_2)_{3-8}-CH_2-CH_2-S-CH_2-CH_2-N(CH_3)_3]^+ CH_3SO_4-$ angegeben wird. Auch ein neutrales Tensid mit der Bezeichnung (R)Zonyl FSB sowie ein basisches ((R)Zonyl FSA) lassen sich sehr gut verwenden.

Als modifizierbare Metallkatalysatoren eignen sich insbesondere Nickel und Edelmetalle wie Palladium und Platin.

Zur Herstellung des modifizierten Katalysators wird das genannte Metall vorzugsweise in hydrierter Form unter einer Wasserstoffatmosphäre in Wasser oder einem Puffer (pro mg Metall 0,2 - 1,0 ml) suspendiert, mit der 2 - 5-fachen Gewichtsmenge (bezogen auf das Metall) an Tensid versetzt und etwa 10 - 30 Stunden bei etwa 20 - 40°C geschüttelt oder gerührt. Anschließend wird der modifizierte Katalysator beispielsweise durch Zentrifugieren oder Dekantieren abgetrennt und unter Sauerstoffausschluß aufbewahrt. Er braucht nicht getrocknet zu werden. In Gegenwart von Sauerstoff wird er langsam inaktiviert.

Durch die Anwesenheit der Tenside werden die Metallkatalysatoren so verändert, daß sie nur die Reduktion der Mediatoren, aber keine Reduktion von ungesättigten Verbindungen und Cosubstraten wie NAD (P) u.a. bewirken. Die Mediatoren werden nicht überreduziert und bleiben daher stabil. Weiter greifen Tensid-Metallkatalysatoren Pyridinnukleotide, die in Redox-Systemen vorliegen können, im Gegensatz zu unmodifizierten Metallkatalysatoren nicht an.

Durch die Verwendung des Katalysators wird es möglich, eine Vielzahl von biochemischen Reduktionen mit Wasserstoff durchzuführen. Die Elektronen des Wasserstoffs werden dabei zunächst auf den Mediator übertragen, der diese anschließend an wasserstoffübertragende Enzyme weitergibt, die ein Substrat reduzieren. Der Katalysator ist nicht in der Lage, den Wasserstoff direkt auf das Substrat zu übertragen. Falls daher bei der Reduktion Verbindungen mit Asymmetriezentren entstehen, wird die Selektivität der Reaktion ausschließlich durch die Enzymspezifität bestimmt.

Das neue Verfahren hat gegenüber den bisherigen Verfahren weiter den Vorteil, daß für die Bildung von reduziertem Mediator weder ein Enzym noch eine elektrochemische Zelle benötigt wird.

Schließlich ist noch zu erwähnen, daß sich das neue System nicht nur zur Regenerierung von isolierten Enzymen, sondern auch von nicht isolierten Enzymen in ganzen Mikroorganismen eignet.

A) Herstellung von modifizierten Katalysatoren

Beispiel 1

a) Modifiziertes Palladium

In 20 ml 0,1 M Phosphatpuffer pH 7,5 wurden 30 mg 10% Palladium auf Kohle (Fa. Merck) mit Stickstoff begast und unter Wasserstoff gesättigt. Nach Zugabe von 120 $\mu$l [R]Zonyl FSC bzw. FSB wurde bei 35°C 10 h mit ca. 120 Ausschlägen/min. unter $H_2$-Atmosphäre geschüttelt. Durch Abzentrifugieren und wieder Suspendieren in gewünschtem Puffer bzw. Volumen kann der Katalysator vom Detergens weitgehend befreit werden. Nach vorliegenden Erfahrungen stört jedoch der Tensidgehalt bei den weiteren Umsetzungen nicht. Für die langfristige Lagerung des modifizierten Katalysators (> 6 Monate) ist Tris . HCl-Puffer besser als Kaliumphosphat.

b) Modifiziertes Platin bzw. Nickel

In 8 ml 0,1 M Kaliumphosphatpuffer pH 7,5 wurden 35 mg feinverteiltes Platin, das durch Hydrierung von Platindioxid erhalten worden war, mit 150 $\mu$l [R]Zonyl FSC bei 35°C 15 h unter Wasserstoffgas geschüttelt. Ebenso wie das Platin wurde durch Auflösen einer Nickel-Aluminium-Legierung mit Natronlauge nach Waschen bis zur Neutralität erhaltenes Nickel mit Wasserstoffgas behandelt.

B) Vergleichsversuche

Beispiel 2

Die folgenden Versuche demonstrieren das veränderte Verhalten von modifiziertem Palladium bzw. Platin gegenüber Methylviologen im Vergleich zu unmodifizierten Katalysatoren:

3,5 mg nach Beispiel 1 modifiziertes Palladium bzw. unmodifiziertes Palladium wurden in getrennten Ansätzen unter Wasserstoff bei 35°C in 3,1 ml 0,1 M Tris-HCl-Puffer bei pH 7,2 in Gegenwart von 30 mM Methylviologen geschüttelt. Im Ansatz mit modifiziertem Palladium waren nach 4 Tagen noch über 98 % des Methylviologens vorhanden, im Ansatz mit dem unmodifizierten Palladium nach 2 Tagen weniger als 1,5 %. Die gleiche Stabilität zeigte 1,1'-Carboxamidmethylviologen.

C) Verwendung des Katalysators

Beispiel 3

a) Herstellung von (2R)-Methyl-buttersäure mit Enoatreduktase, Wasserstoffgas und modifiziertem Palladium

12 mg nach Beispiel 1 modifiziertes Palladium wurden in 10 ml Kaliumphosphatpuffer pH 7,0 zusammen mit 0,04 mMol Methylviologen-hydrochlorid, 0,8 mMol Natriumsalz von (E)-2-Methylbutenoat und 2 U Enoatreduktase unter einer $H_2$-Atmosphäre bei Normaldruck und bei 27°C gerührt. Nach ca. 15 h betrug der Umsatz über 98 %. Ein Teil des Produkts wurde nach Extraktion mit Diethylether mit (R)-Phenylglycinol zum entsprechenden Amid umgesetzt. Nach HPLC-Analyse betrug die Enantiomerenreinheit des Amids (ee) über 97 %.

Läßt man die Enoatreduktase weg, so gelingt die Umsetzung nicht.

Führt man die Reaktion ohne Enoatreduktase aber in Gegenwart von nicht modifiziertem Palladium durch, so erhält man racemische Methylbuttersäure.

b) Analog wurde (2R)-2-Methyl-3-phenylpropionsäure aus (E)-2-Methylzimtat mit gleicher Ausbeute und Enantiomerenreinheit erhalten.

Beispiel 4

Reduktion von Zimtat zu 3-Phenylpropionat mit modifiziertem Platin, Wasserstoffgas und Enoatreduktase.

Zu 3,0 ml 0,12 M Tris-HCl-Puffer pH 7,0 wurden 7,5 mg modifiziertes Platin, 3,3 mM Methylviologen und 70 mM Zimtat gegeben. Das System wurde bei 35°C unter $H_2$-Atmosphäre geschüttelt. Die Lösung wurde rasch blau. Es wurden nur so viel Wasserstoff verbraucht, wie für die Reduktion des Methylviologens benötigt werden, d.h. ca. 5 $\mu$mol. Danach wurde für 4 h kein Wasserstoffverbrauch mehr beobachtet. Anschließend wurden 1,3 U Enoatreduktase zugegeben und wieder Wasserstoff eingeleitet, was zur quantitativen Reduktion des Zimtats in 5 h führte. Die Lösung wurde noch für weitere 10 Tage geschüttelt, wobei das Methylviologen unverändert blieb.

Beispiel 5

(2R)-2-Hydroxy-4-methylpentanoat mit partiell gereinigter 2-Oxosäurereduktase, Wasserstoffgas und modifiziertem Palladium
30 mg modifiziertes Palladium wurde in 50 ml 0,1 M Phosphatpuffer pH 7,0 zusammen mit 0,2 mMol Methylviologen, 5 mMol 2-Oxo-4-methylpentanoat und 8 U partiell gereinigter 2-Oxosäurereduktase unter $H_2$-Atmosphäre bei 25° C geschüttelt. Nach dem der Umsatz nach 40 h bei über 98 % lag, wurde das (2R)-2-Hydroxy-4-methylpentanoat isoliert und die optische Drehung mit

$$[\alpha]_{RT}^{589} = +10,9°$$

(c = 22 mg/ml) bestimmt. Dies entspricht einem ee-Wert von über 95 %.

Beispiel 6

Herstellung von (2R)-Propandiol mit Hefe, Wasserstoffgas und modifiziertem Palladium
7 mg nach Beispiel 1 modifiziertes Palladium wurden in 10 ml 0,1 M Phosphatpuffer pH 7,3 mit 0,03 mMol Methylviologen, 0,01 mMol NAD, 140 mg Candida utilis (DSM 70 167), 1 mMol Hydroxyaceton unter $H_2$-Atmosphäre bei 25° C gerührt. Nach 9 h waren bei entsprechendem Wasserstoffverbrauch 90 % Propandiol gebildet.
Ein entsprechender Versuch ohne Zusatz von NAD dauerte 13 h, lieferte aber dieselbe Ausbeute.

**Patentansprüche**

1. Verfahren zur selektiven Regenerierung von Mediatoren durch katalytische Hydrierung, dadurch gekennzeichnet, daß man als Katalysator einen mit Hilfe von Tensiden modifizierten Metallkatalysator verwendet.

2. Verwendung der Katalysatoren gemäß Anspruch 1 zur Durchführung biochemischer Reduktionen.

**Claims**

1. A process for the selective regeneration of a mediator by catalytic hydrogenation, wherein the catalyst used is a metal catalyst modified with the aid of a surfactant.

2. Use of a catalyst as claimed in claim 1 for carrying out biochemical reductions.

**Revendications**

1. Procédé pour la régénération sélective de médiateurs par hydrogénation catalytique, caractérisé en ce que l'on utilise, en tant que catalyseur, un catalyseur métallique modifié à l'aide d'agents tensio-actifs.

2. Utilisation des catalyseurs selon la revendication 1 pour l'exécution de réductions biochimiques.